# EUROPEAN PATENT APPLICATION

(11) **EP 4 560 004 A1**
(43) Date of publication of application: **28.05.2025**
(21) Application number: 23841961.8
(22) Date of filing: 12.06.2023
(51) Int. Cl.: C12M 1/34, C12M 1/00, B04B 5/04, B04B 15/02, B04B 9/02, G01G 17/00, G01G 21/10

(54) **CELL TREATMENT DEVICE**

(30) Priority: 22.07.2022 CN 202210870017
(71) Applicant: Shenzhen Cellbri Bio-Innovation Technology Co., Ltd., Shenzhen, Guangdong 518107 (CN)
(72) Inventor: FANG, Rulin, Shenzhen, Guangdong 518107 (CN); CHEN, Zhilin, Shenzhen, Guangdong 518107 (CN); ZHU, Junshu, Shenzhen, Guangdong 518107 (CN); ZHAO, Huimin, Shenzhen, Guangdong 518107 (CN); SHANG, Yuanfang, Shenzhen, Guangdong 518107 (CN); GUO, Xiaoliang, Shenzhen, Guangdong 518107 (CN)
(74) Representative: Herrero & Asociados, S.L.
(86) International application number: PCT/CN2023/099698
(87) International publication number: WO 2024/016895

(57) **Abstract**

The application belongs to the technical field of cell processing, in particular to a cell processing device. The weighing device of the cell processing device is provided with at least one set of weighing components. These components allow one or more samples hung on the hook to be weighed and detected simultaneously, increasing weighing efficiency. The samples hung on each hook are weighed using the designated weighing sensor, so the weighing results won't interfere with one another. Moreover, each set of weighing components in this application can be disassembled and reassembled, making overhaul and maintenance easier. Furthermore, in the application, the centrifugal container is installed via a centrifuge module specifically designed for the centrifugal container, which drives the centrifugal container to rotate, making the centrifugal container installation more convenient and firm, avoiding the situation in which the centrifugal container falls off, and ensuring the safety of the cell processing process. The present application has the advantages of simple structure, reduced device volume and convenient maintenance.

## Description

This application claims priority to Chinese Patent Application No. 202210870017.7, filed on July 22, 2022, titled "Cell processing device", the contents of which are incorporated herein by reference in their entirety.

### Technical field

The application belongs to the technical field of cell processing, in particular to a cell processing device.

### Background

At present, cell processing devices are mostly used for cell processing, such as separation or mixing. In the prior art, the cell processing device typically had a single function and a huge device volume. The inventors discovered that in the process of cell processing by cell processing device, the cell processing device can only weigh a group of samples to be processed by weighing device at a time, resulting in low weighing efficiency. Furthermore, the weighing device has a large volume, and when a fault occurs, the troubleshooting process is cumbersome, which is unsuitable for maintenance.

### Summary of the invention

To address the technical issues of single function and low weighing efficiency of the cell processing device in the prior art, the application provides a cell processing device.

In view of the above technical problems, an embodiment of the present application provides a cell processing device, including:
a weighing device, comprising a support component and at least one set of weighing components, wherein the support component comprises a bracket for supporting the weighing component; each set of weighing components comprises a mounting support seat installed on the bracket, a weighing sensor installed on the mounting support seat, and a hook detachably installed on the weighing sensor for hanging a sample;
a centrifugal device, comprises a centrifugal bowl with a centrifugal cavity, a centrifugal container arranged in the centrifugal cavity, and a centrifuge module connected with the centrifugal bowl and used for driving the centrifugal container to rotate;
a liquid path module, comprises a conduit component for conveying liquid in the sample into the centrifugal container for cell processing, and a liquid path panel for installing and controlling a liquid flow in the conduit component; and
a housing, wherein the centrifugal bowl, the liquid path panel and the bracket are all installed on the housing.

Meanwhile, the samples hung on each hook are weighed using the designated weighing sensor, so the weighing results won't interfere with one another. Moreover, each set of weighing components in this application can be disassembled and reassembled, making overhaul and maintenance easier.

For the cell processing device of the application, the weighing device of the cell processing device is provided with at least one set of weighing components. These weighing components allow one or more samples hung on the hook to be weighed and detected simultaneously, increasing weighing efficiency. Meanwhile, the weighing component has a hook for hanging the sample, which makes it easy to hang the sample and does not impede liquid flow in the hung sample. In addition, the weighing results of each sample will be separate from and unaffected by the weighing results of the others when multiple samples are weighed simultaneously since the samples hung on each hook are weighed by the designated weighing sensor. Moreover, each set of weighing components in this application is installed on the bracket through the mounting support seat, so that the disassembly and installation of a single set of weighing components can be realized, and the overhaul and maintenance are convenient. Furthermore, in the application, the centrifugal container is installed via a centrifuge module specifically designed for the centrifugal container, which drives the centrifugal container to rotate, making the centrifugal container installation more convenient and firm, avoiding the situation in which the centrifugal container falls off, and ensuring the safety of the cell processing process. The cell processing device in the application has the advantages of simple structure, reduced device volume; and each part is separately and detachably installed on the housing. This simplifies the process of troubleshooting when a fault occurs and is convenient for maintenance.

Details of one or more embodiments of the present application are set forth in the following drawings and description. Other features and advantages of this application will be apparent from the description, drawings and claims.

### Brief description of drawings

The application will be further illustrated with the embodiments and attached drawings.
Fig. 1 is a structural schematic diagram of a cell processing device provided by an embodiment of the present application.
Fig. 2 is an exploded view of the structure of a weighing device of a cell processing device provided by an embodiment of the present application.
Fig. 3 is an exploded view of the structure of a weighing component of a cell processing device provided by an embodiment of the present application.
Fig. 4 is a structural schematic diagram of a hook of a cell processing device provided by an embodiment of the present application.
Fig. 5 is a structural schematic diagram of a hook of a cell processing device provided by another embodiment of the present application.
Fig. 6 is a schematic diagram of the assembly structure of a centrifuge module of a cell processing device provided by an embodiment of the present application.
Fig. 7 is an exploded view of the structure of a centrifuge module of a cell processing device provided by an embodiment of the present application.
Fig. 8 is an exploded view of the structure of a centrifuge module and a temperature control module of a cell processing device provided by an embodiment of the present application.
Fig. 9 is a schematic diagram of the assembly structure of a centrifuge module and a temperature control module of a cell processing device provided by an embodiment of the present application.
Fig. 10 is a structural schematic diagram of a liquid path module of a cell processing device provided by an embodiment of the present application.

The reference numerals in the specification are as follows:
1. Weighing device; 11. Support component; 111. Bracket; 1111. Mounting plate; 1112. Mounting hole; 1113. Support bar; 112. Front housing; 113. Back housing; 12. Weighing component; 121. Mounting support seat; 1211. Accommodation groove; 1212. Fixing hole; 1213. Flange; 122. Weighing sensor; 123. Hook; 1231. Connecting thread; 1232. Bending section; 1233. Lateral deflection section; 1234. Hitch section; 12341. Connecting part; 1235. Opening; a. Preset lateral deflection angle; l1. Central axis of the hitch section; 124. Front cover; 125. Back cover;
2. Centrifugal device; 21. Centrifugal bowl; 212. Connecting flange; 22. Temperature control module; 221. Refrigeration component; 2211. first semiconductor chilling plate; 2212. Second semiconductor chilling plate; 222. Cooling component; 2221. Refrigeration fan; 2222. Cooling plate; 2223. First air conduit; 2224. Mounting part; 2225. Positioning groove; 2226. Cooling fan; 2227. Second air conduit; 2228. Heat conduit cooler; 223. Heat preservation layer; 224. Temperature sensor; 23. Liquid discharging connector; 24. Sealing ring; 25. Liquid detection sensor; 26. Flip cover;
100. Centrifuge module; 101. Rotating shaft; 1011. Positioning step; 1012. Pin; 1013. Pin hole; 102. Bearing component; 1021. Bearing seat; 1022. Bearing; 103. Coupler; 104. Drive motor; 105. Actuator; 1051. Via hole; 106. Rotating mounting seat; 107. Irregular wheel; 108. Motor mounting seat; 1081. Motor accommodation space; 109. Fixing plate;
3. Liquid path module; 31. Conduit component; 311. Pinch valve; 312. Bubble sensor; 313. Pressure sensor; 314. Peristaltic pump; 315. Stepping motor; 32. Liquid path panel;
4. Sample; 5. Housing; 51. Start button; 6. Code scanning device; 7. Display device.

### Detailed description of disclosed embodiments

In order to make the technical problems, technical solutions and beneficial effects of the present application more clear, the application will be further explained in detail below with reference to the drawings and embodiments. It should be understood that the specific embodiments described here are only used to illustrate the application, rather than to limit the application.

It should be understood that the terms "upper", "lower", "left", "right", "front", "back", "middle", and the like indicate an orientation or positional relationship based on that shown in the drawings, and are for convenience of description and simplicity of description only, not intended to indicate or imply that the indicated devices or elements must have a particular orientation, be constructed and operated in a particular orientation, and are therefore not to be construed as limiting the present application.

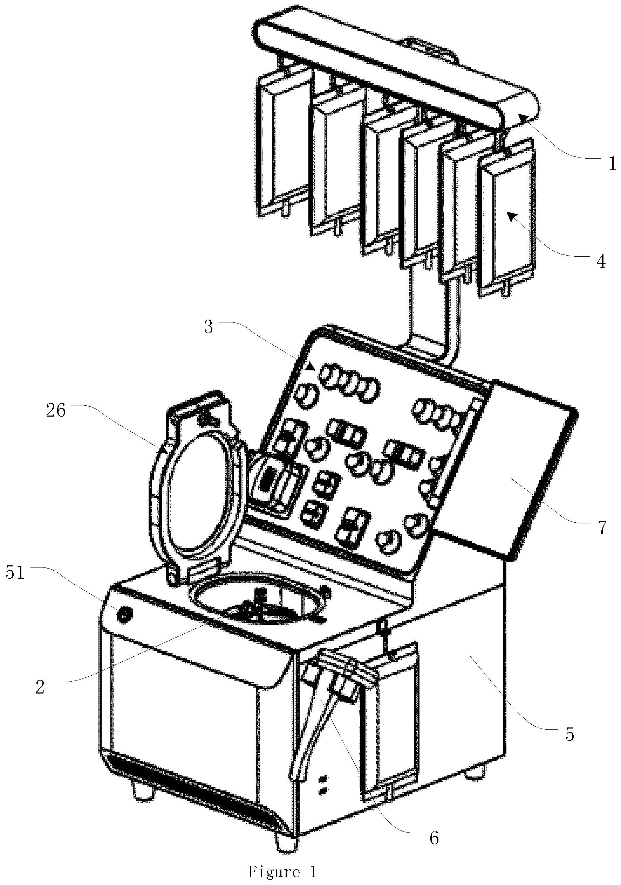

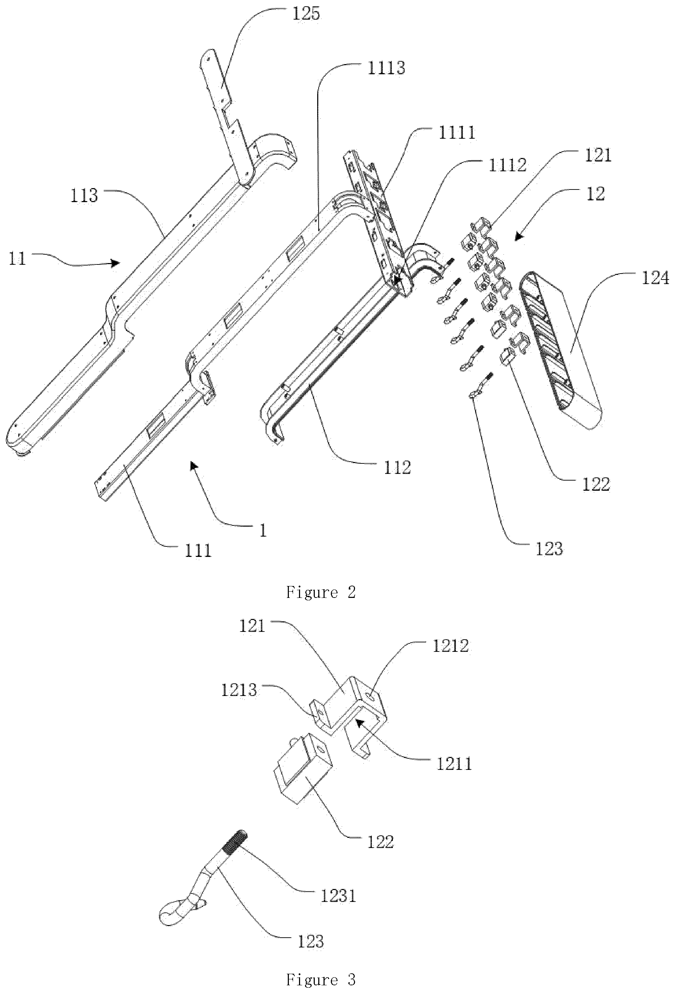

As shown in Fig. 1 and Fig. 2, an embodiment of the present application provides a cell processing device, including the following elements.

A weighing device 1, including a support component 11 and at least one set of weighing components 12; the support component 11 comprises a bracket 111 for supporting the weighing component 12; each set of weighing components 12 includes a mounting support seat 121 installed on the bracket 111, a weighing sensor 122 installed on the mounting support seat 121, and a hook 123 detachably installed on the weighing sensor 122 to hang a sample 4 (such as a liquid bag for holding liquid). The weighing sensor 122 may be a pressure sensor, which determines the weight through a pressure signal. Both the bracket 111 and the mounting support seat 121 may be sheet metal parts, so that the bracket 111 is firmer, and better load bearing can be realized. Meanwhile, the sheet metal part of mounting support seat 121 is not easy to deform and can better protect the weighing sensor 122, etc. The shape and size of the bracket 111 can be designed as required, as long as the weighing component 12 can be stably installed on the bracket 111 and the volume of the whole weighing device 1 can be reduced as much as possible. In the above embodiment of the application, one or more sets of weighing components 12 with the same structure are provided in the weighing device 1 (the number of weighing components 12 can be determined according to requirements). Each set of weighing components 12 is installed on the bracket 111 through the mounting support seat 121, allowing the disassembly and installation of the single set of weighing components 12. And if a single set of weighing components 12 is damaged, the use of other sets of weighing components 12 would not be affected. Meanwhile, because each set of weighing components 12 has the same structure, it is convenient for overhaul and maintenance. Understandably, the hook 123 may also be designed to be detachably connected to the weighing sensor 122, which is convenient to disassemble and install the hook 123. Moreover, it can improve the efficiency of troubleshooting. In an embodiment, the weighing sensor 122 is provided with a threaded hole (not shown), and the hook 123 is provided with a connecting thread 1231, and the hook 123 is in threaded connection with the threaded hole through the connecting thread 1231. That is, in this embodiment, the detachable connection between the hook 123 and the weighing sensor 122 is realized through the threaded connection between the connecting thread 1231 and the threaded hole.

Further, as shown in Figs. 2 and 3, the mounting support seat 121 is provided with an accommodation groove 1211 adapted to the weighing sensor 122, and a fixing hole 1212 is provided on the inner wall of the accommodation groove 1211. The weighing sensor 122 is fixedly installed in the accommodation groove 1211 by a screw passing through the fixing hole 1212. That is, the mounting support seat 121 may be a bending plate bent to form the accommodation groove 1211, and the weighing sensor 122 is installed in the accommodation groove 1211, which can be protected from damage by the mounting support seat 121. Since the lower end of the weighing sensor 122 needs to be connected with the hook 123, the fixing hole 1212 in this embodiment is arranged on the top inner wall of the accommodation groove 1211, so that the top of the weighing sensor 122 is fixed with the mounting support seat 121 and is not easy to be detached from the accommodation groove 1211. Optionally, the mounting support seat 121 includes a flange 1213 arranged at the edge of the accommodation groove 1211, and the mounting support seat 121 is installed on the bracket 111 through the flange 1213. Specifically, the mounting support seat 121 can be fixed on the mounting plate 1111 through the flange 1213 mentioned above, and the connection mode between the mounting support seat 121 and the mounting plate 1111 may be detachable, such as screw connection and snap connection. This allows the mounting support seat 121 and the whole weighing component 12 to be detached from the mounting plate 1111.

A centrifugal device 2, comprising a centrifugal bowl 21 with a centrifugal cavity, a centrifugal container (not shown) arranged in the centrifugal cavity, and a centrifuge module 100 connected with the centrifugal bowl 21 and used for driving the centrifugal container to rotate. The centrifuge module 100 of the centrifugal device 2 drives the centrifugal container positioned in the centrifugal cavity of the centrifugal bowl 21 to rotate, so as to allow cell processing performed on the liquid injected in the centrifugal container. In this embodiment, the centrifugal container is installed via a centrifuge module 100 specifically designed for the centrifugal container, which drives the centrifugal container to rotate, making the centrifugal container installation more convenient and firm, avoiding the situation in which the centrifugal container falls off, and ensuring the safety of the cell processing process.

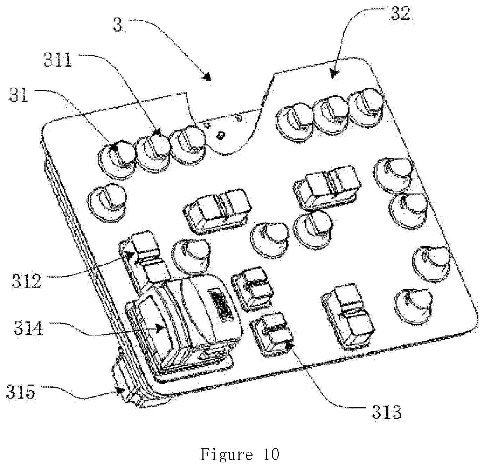

A liquid path module 3, comprising a conduit component 31 for conveying the liquid in the sample 4 into the centrifugal container for cell processing, and a liquid path panel 32 for installing and controlling the liquid flow in the conduit component 31. That is, for the liquid path module 3, as shown in Fig. 10, the conduit component 31 is installed on the liquid path panel 32, and by operating and controlling the conduit component 31 on the liquid path panel 32, the liquid in the sample 4 can smoothly flow into the centrifugal container from the conduit component 31 for cell processing.

A housing 5, the centrifugal bowl 21, the liquid path panel 32 and the bracket 111 are all installed. Understandably, the shape of the housing 5 can be designed as required, and it is not limited by Fig. 1. The housing 5 includes a support frame, a bottom plate installed at the bottom of the support frame, a side plate installed around the support frame, and a top plate installed at the top of the support frame. The above-mentioned bottom plate, top plate and side plate can be assembled together by means of screw connection, snap connection or welding, etc. The bottom plate, top plate and side plate can be detachably connected with the support frame, so as to facilitate the installation and maintenance of components inside the housing 5 at the disassembly position (such as conduit insertion, replacement of centrifugal containers, etc.).

The weighing device 1 of the cell processing device in the application is provided with at least one set of weighing components 12, and one or more samples 4 hanging on the hook 123 can be simultaneously weighed and detected by the weighing components 12, thus improving the weighing efficiency. Meanwhile, the weighing component has a hook 123 for hanging the sample 4, which makes it easy to hang the sample 4 and does not impede liquid flow in the hung sample. In addition, the weighing results of each sample 4 will be separate from and unaffected by the weighing results of the others when multiple samples are weighed simultaneously since the samples hung on each hook 123 are weighed by the designated weighing sensor 122. Moreover, each set of weighing components 12 in this application is installed on the bracket 111 through the mounting support seat 121, so that the disassembly and installation of a single set of weighing components 12 can be realized, and the overhaul and maintenance are convenient. Furthermore, in the application, the centrifugal container is installed via a centrifuge module 100 specifically designed for the centrifugal container, which drives the centrifugal container to rotate, making the centrifugal container installation more convenient and firm, avoiding the situation in which the centrifugal container falls off, and ensuring the safety of the cell processing process. The cell processing device in the application has the advantages of simple structure, reduced device volume; and each part is separately and detachably installed on the housing 5. This simplifies the process of troubleshooting when a fault occurs and is convenient for maintenance.

In an embodiment, as shown in Figs. 2 and 3, there are at least two sets of weighing components 12. The bracket 111 includes a mounting plate 1111 provided with mounting holes 1112 at intervals (preferably at uniform intervals), and the number of the mounting holes 1112 is the same as that of the weighing components 12. The mounting support seat 121 is used to install the weighing sensor 122 on the mounting plate 1111 at a position opposite to the mounting hole 1112, and the hook 123 passes through the mounting hole 1112 to connect with the weighing sensor 122. In this embodiment, each mounting hole 1112 is used to install a set of weighing components 12. Understandably, the mounting hole 1112 may also be designed as a groove, as long as the hook 123 can pass through to connect with the weighing sensor 122, and the installation of the mounting support seat 121 on the mounting plate 1111 is ensured to be stable and reliable. Further, the bracket 111 also includes a support bar 1113 connected to the mounting plate 1111, and the support component 11 also includes a front housing 112 and a back housing 113, and the front housing 112 and the back housing 113 enclose to form an installation space for installing the support bar 1113. That is, the support bar 1113 is used to support the mounting plate 1111 and all the weighing components 12 installed on the mounting plate 1111 to keep the weighing components 12 stable during weighing. And the front housing 112 and back housing 113 are used to protect the support bar 1113. In an embodiment, the weighing component 12 further comprises a front cover 124 and a back cover 125, and the front cover 124 and the back cover 125 enclose to form an accommodation space for accommodating the mounting plate 1111, the mounting support seat 121 and the weighing sensor 122. That is, after the weighing component 12 is installed on the mounting plate 1111, the front cover 124 and the back cover 125 further protect the mounting plate 1111 and the weighing sensor 122 in the weighing component 12 in the accommodating space to prevent them from being damaged.

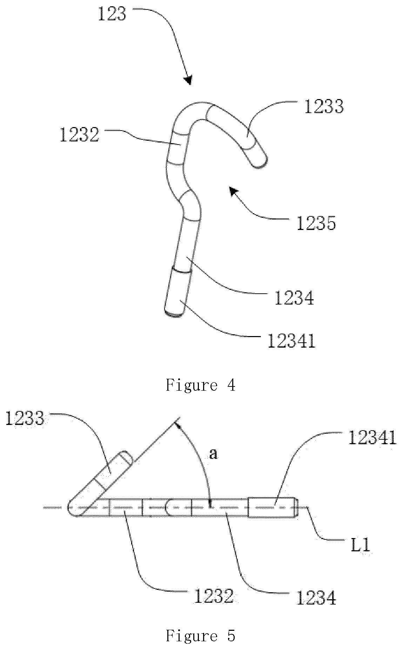

Further, as shown in Figs. 4 and 5, the hook 123 includes a bending section 1232, a lateral deflection section 1233, and a hitch section 1234 (understandably, the connecting thread 1231 may be an external thread arranged on the hitch section 1234). The opposite ends of the bending section 1232 are respectively connected with the hitch section 1234 and the lateral deflection section 1233. The bending section 1232 and the lateral deflection section 1233 enclose to form a hook ring with an opening 1235. The bending section 1232 and the central axis of the hitch section L1 are both positioned on a first preset datum plane, and the central axis of the lateral deflection section 1233 is arranged at a preset lateral deflection angle (a) with the first preset datum plane. In an embodiment, the bending section 1232, the lateral deflection section 1233 and the hitch section 1234 are integrally formed, which can simplify the manufacturing process of the hook 123, improve the manufacturing efficiency and reduce the manufacturing cost. In some embodiments, the detachable connection among the bending section 1232, the lateral deflection section 1233 and the hitch section 1234 is also possible, which is convenient for storage and transportation. If a part of the bending section 1232 is damaged and needs to be replaced, simply replace the damaged part. Understandably, the hook 123 can be made of metal, such as stainless steel. Understandably, the bending shape and bending angle of the bending section 1232 can be designed as required. In this embodiment, the bending section 1232 and the central axis of the hitch section L1 are both positioned in the same plane (i.e., both are positioned in the first preset datum plane, further, when the hook 123 is detachably connected to the weighing sensor 122, the first preset datum plane is perpendicular to the horizontal plane). In some embodiments, the central axis of the bending section 1232 may be partially located in the first preset datum plane, and the other part may be bent and extended out of the first preset datum plane. Understandably, the shape of the hook ring changes with the shapes of the lateral deflection section 1233 and the bending section 1232, which is not limited in this application. When it needs to hang the sample 4 into the hook ring, the sample 4 will be hung from the opening 1235 of the hook ring. Specifically, the connection point between the lateral deflection section 1233 and the bending section 1232 is located on the first preset datum plane. From this connection point, the lateral deflection section 1233 laterally deflects in the direction away from the first preset datum plane. Understandably, the distance between the lateral deflection section 1233 and the first preset datum plane gradually increases in the direction from the connection point to the end away from the bending section 1232. It should be noted that in this application, the lateral deflection section 1233 may not necessarily be a straight line, but a curve (the central axis of the lateral deflection section 1233 may be fitted as a straight line according to its bending direction, etc.), as long as it deflects in the direction away from the first preset datum plane and the deflection angle reaches a preset deflection angle.

In this embodiment, the sample 4 (not shown) can be hung forward from the opening 1235 position of the hook ring (the wider side of the sample 4 faces the console of the cell processing device) into the hook ring. And since the central axis of the lateral deflection section 1233 is arranged at a preset lateral deflection angle (a) with the first preset datum plane, when the sample 4 falls to the bottom of the hook ring along the lateral deflection section 1233 by its own gravity, the sample 4 will naturally be hung laterally (the narrower side of sample 4 faces the console) on the hook 123, thus reducing the lateral width of sample 4 arranged on the support component 11 of the cell processing device, and further hanging more samples 4 on the same support component 11, which improves the operation efficiency. And in the process of hanging the sample 4 through the hook 123 of the application, the user can directly hang the sample 4 from the front to obtain the laterally arrange sample 4, which saves time and labor and greatly improves the user's operation experience.

Further, the preset lateral deflection angle (a) is 30-60 degrees. Preferably, the preset lateral deflection angle (a) is 45 degrees. The preset lateral deflection angle (a) is set to fall into the above-mentioned angle range. This allows the user to hang the sample 4 from the front where the console is positioned, thus improving the operation convenience. Meanwhile, the sample 4 hanging in the front can fall along the lateral deflection section 1233 in the hook ring by its own gravity to realize lateral arrangement, thereby reducing the lateral width of the sample 4 arranged on the support component 11 of the cell processing device, enabling more samples 4 to be hung on the same support component 11, and improving the operation efficiency.

In an embodiment, as shown in Figs. 4 and 5, the central axis of the hitch section L1 passes through the connection point between the bending section 1232 and the lateral deflection section 1233. A first relative distance is greater than or equal to a second relative distance. The first relative distance refers to the relative distance between the connection point and a second preset datum plane. The second preset datum plane is perpendicular to the hitch section 1234 and passes through any point on the hitch section 1234. The second relative distance refers to the relative distance between other points on the bending section 1232 and the lateral deflection section 1233 except the connection point and the second preset datum plane. Understandably, after the hook 123 is installed on the cell processing device, the hitch section 1234 and its first preset datum plane are perpendicular to the horizontal plane. Because the second preset datum plane is perpendicular to the hitch section 1234, the second preset datum plane is a plane parallel to the horizontal plane at this point, and the second datum plane passes through any point on the hitch section 1234. For example, the second datum plane passes through the adjacent point between the hitch section 1234 and the bending section 1232. At this state, since the first relative distance is greater than or equal to the second relative distance, the connection point between the bending section 1232 and the lateral deflection section 1233 is the lowest point of the current installation state of the whole hook 123. Therefore, after the sample 4 is hung on the hook 123 from the position of the opening 1235 of the hook ring and naturally falls by its own gravity, it will fall to the connection point between the bending section 1232 and the lateral deflection section 1233, and the connection point is located directly below the central axis of the hitch section L1. In this way, after the sample 4 slides to the connection point, the hitch section 1234 of the hook 123 will remain vertical to the horizontal plane, and will not deflect or shake under the gravity of the sample 4, thus ensuring the stability of the cell processing device. Optionally, the bending section 1232 includes a straight line segment parallel to the central axis of the hitch section L1. The above arrangement can make the structure of the hook 123 more stable and reliable.

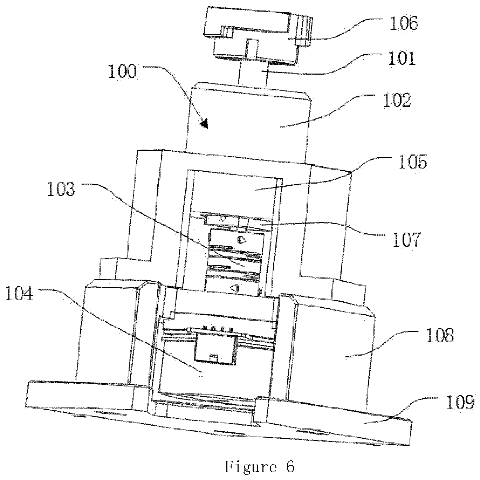

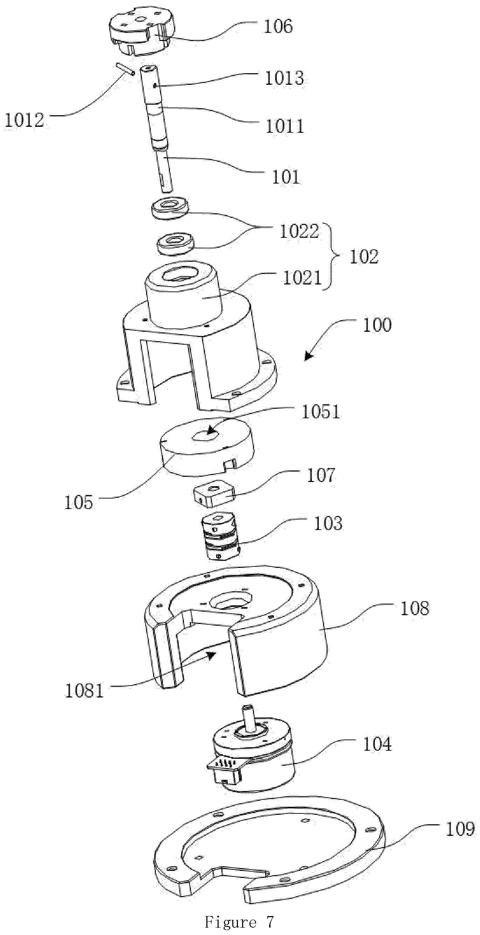

In an embodiment, as shown in Figs. 6 and 7, the centrifuge module 100 includes a rotating shaft 101, a bearing component 102, a coupler 103, a drive motor 104 for driving the rotating shaft 101 to rotate, an actuator 105 for actuating the rotating shaft 101, and a rotating mounting seat 106 positioned in the centrifugal cavity and used for installing the centrifugal container. The coupler 103 is connected between the output end of the drive motor 104 and the rotating shaft 101. An end of the rotating shaft 101 away from the coupler 103 passes through the bearing component 102 and is connected with the rotating mounting seat 106. The actuator 105 is connected with the bearing component 102. The rotating mounting seat 106 is installed on the rotating shaft 101 via a pin 1012. Specifically, the rotating shaft 101 is provided with a pin hole 1013 at one end away from the coupler 103. The rotating mounting seat 106 is clamped into the pin 1012 passing through the pin hole 1013, and then fixed on the rotating shaft 101 by screws. Further, the rotating mounting seat 106 is provided with a first clamping part, and the centrifugal container is provided with a second clamping part. The centrifugal container is connected with the rotating mounting seat 106 through the clamping of the first clamping part and the second clamping part. Further, after clamping, the centrifugal container and the rotating mounting seat 106 can be further fixedly connected through screw components to make the connection between them stable. The rotating mounting seat 106 allows the centrifuge module 100 to be compatible with centrifugal containers of various specifications. Further, the drive motor 104 is a brushless DC motor, so as to prolong the motor service life of the centrifuge module 100 and reduce the noise. Further, the coupler 103 is a diaphragm coupler. Since diaphragm coupler has no backlash, the transmission accuracy of centrifuge module 100 can be further improved by using diaphragm coupler. Understandably, the actuator 105 actuates the rotating shaft 101 through the actuator disc when power is cut off. The actuator 105 releases the actuator disc to terminate the actuation state of the rotating shaft 101 under the power-on condition. At this point, the drive motor 104 can operate to drive the rotating shaft 101 to rotate through the coupler 103, the rotating shaft 101 would drive the centrifugal container installed on rotating mounting seat 106 to rotate, thus realizing cell processing such as separation, mixing, preparation and cleaning of sample 4 (such as blood) in the centrifugal container.

In this embodiment, the rotating shaft 101 is rotatably installed on the bearing component 102 to limit the radial runout range of the rotating shaft 101 and reduce the radial runout when the centrifuge module 100 drives the centrifugal container to rotate. Moreover, the rotating shaft 101 is connected with the rotating shaft 101 and the drive motor 104 through the coupler 103, and the rotating shaft 101 is actuated and driven by the actuator 105 and the drive motor 104 respectively, so that the structure is simple and the transmission accuracy is improved. In addition, in this application, the rotating mounting seat 106 installed on the rotating shaft 101 is specially adapted to install the centrifugal container, which makes the installation of the centrifugal container more convenient and firm, avoids the situation that the centrifugal container falls off when the rotating shaft 101 drives the centrifugal container to rotate through the rotating mounting seat 106, and ensures the safety of the cell processing process, as well as the reliable quality and better effect of the cell processing.

Further, as shown in Fig. 7, the bearing component 102 includes a bearing seat 1021 with a mounting through hole and two bearings 1022 mounted on the inner side wall of the mounting through hole. The rotating shaft 101 passes through the two bearings 1022 and is installed on the bearing seat 1021. The actuator 105 is installed on the bearing seat 1021. The actuator 105 is provided with a via hole 1051, and the rotating shaft 101 is rotatably connected with the bearing seat 1021 through the two bearings 1022 after passing through the via hole 1051. Further, the actuator 105 is fixedly installed on the bearing seat 1021 by screws, and the outer rings of the two bearings 1022 are installed on the inner side wall of the mounting through hole in the bearing seat 1021 in an interference fit manner. Optionally, the inner side wall of the through hole is recessed to form two annular grooves (not shown) arranged in parallel. The two bearings 1022 are respectively clamped in the two annular grooves, and the rotating shaft 101 passes through the via hole 1051 on the actuator 105 and then passes through the two bearings 1022 to be rotationally connected with the bearing seat 1021 through the two bearings 1022. Optionally, the rotating shaft 101 is provided with a positioning step 1011, and two bearings 1022 are installed on the positioning step 1011 to stably fix the inner ring of the bearing 1022 on the positioning step 1011. In this embodiment, the bearing components 102 of the two bearings 1022 is used to limit the position of the rotating shaft 101, which limits the radial runout range of the rotating shaft 101 to the greatest extent.

Further, as shown in Figs. 6 and 7, the centrifuge module 100 further includes an irregular wheel 107 installed on the rotating shaft 101, and the actuator 105 is provided with a clamping groove (not shown) adapted to the irregular wheel 107. Understandably, the shape of the irregular wheel 107 can be designed according to requirements. For example, the irregular wheel 107 may be a square wheel, and at this point, the clamping groove will be designed as a square groove adapted to the square wheel. Before the actuator 105 starts, the irregular wheel 107 is disengaged from the clamping groove, and the square groove will not actuate the rotating shaft 101 connected with the square wheel. While after the actuator 105 actuates, the rotating shaft 101 is actuated by the actuator disc at first, and then the rotating speed of the rotating shaft 101 is reduced, and the square wheel will also be clamped in the groove with the axial movement of the rotating shaft 101, so that the rotating shaft 101 will stop moving quickly.

In an embodiment, as shown in Figs. 6 and 7, the centrifuge module 100 further includes a motor mounting seat 108 with a motor accommodation space 1081, and the drive motor 104 is installed in the motor accommodation space 1081. The bearing component 102 is installed on the motor mounting seat 108. Specifically, in this embodiment, the drive motor 104 is installed and placed through the motor accommodation space 1081 formed by the sinking platform provided on the motor mounting seat 108, and the inner side wall of the motor accommodation space 1081 is provided with a central shaft hole. The output end of the motor (i.e., the output shaft) passes through the central shaft hole and is connected with the coupler 103. After that, the drive motor 104 will be fixedly installed (such as connected by screws) in the sinking platform. Further, the centrifuge module 100 also includes a fixing plate 109 for connecting an external component, and an end of the motor mounting seat 108 away from the bearing seat 1021 is mounted on the fixing plate 109. That is, the opening end of the sinking platform on the motor mounting seat 108 (the opening end of the motor accommodation space 1081) is connected and fixed with the fixing plate 109. Furthermore, the fixing plate 109 can be used to fix the whole centrifuge module 100 on the external component, for example, at the bottom of the centrifugal cavity of the centrifugal bowl 21 of the cell processing device, so that the centrifuge module 100 can drive the centrifugal container to perform cell processing operation in the centrifugal cavity.

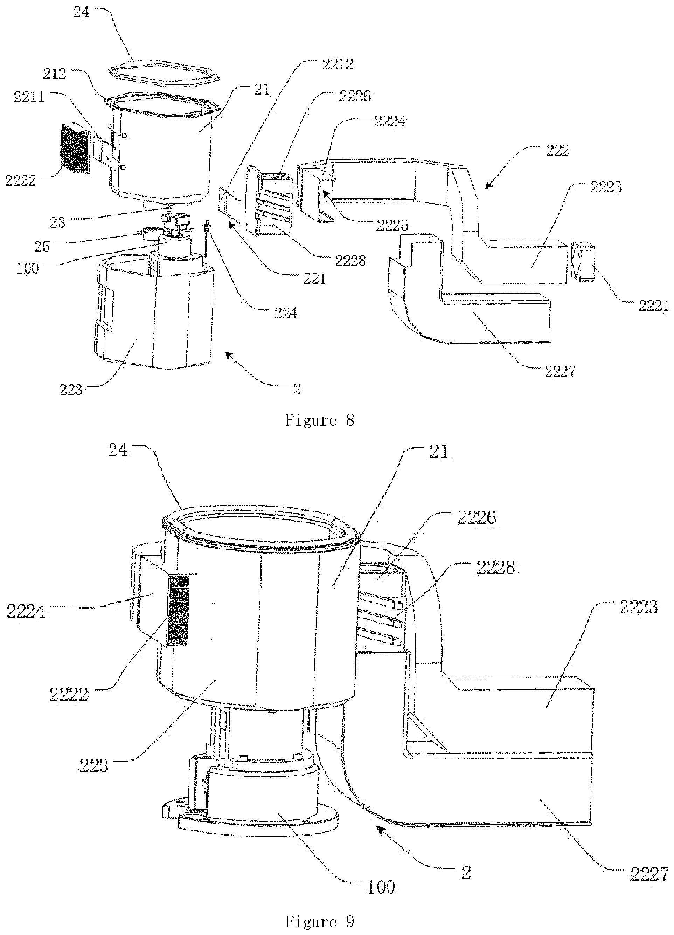

In an embodiment, as shown in Figs. 8 and 9, the centrifugal device 2 further includes a temperature control module 22 for controlling the temperature of the centrifugal cavity. The temperature control module 22 includes a refrigeration component 211 for chilling the centrifugal cavity, and a cooling component 222 for cooling the refrigeration component 211. The chilling surface of the refrigeration component 211 is attached to the outer side wall of the centrifugal bowl 21, and the heating surface of the refrigeration component 211 is connected with the cooling component 222. The refrigeration component 211 may be at least one semiconductor chilling plate with a chilling surface attached to the inner side wall of the centrifugal bowl 21, and the electrified semiconductor chilling plate can chill the centrifugal cavity inside the centrifugal bowl 21 through the chilling surface, so that the cooling speed is high and the cooling efficiency is high. The refrigeration component 211 may also be a peltier, and the refrigeration component 211 can also realize a heating function. That is, the refrigeration component 211 realizes a working mode contrary to the refrigeration mode. The chilling surface heats, and the surface opposite to the chilling surface chills, thereby achieving the effect that the refrigeration component 211 provides heat. The centrifuge module 100 of centrifugal device 2 drives the centrifugal container positioned in the centrifugal cavity of the centrifugal bowl 21 to rotate, so as to perform cell processing. The temperature control module 22 can chill the centrifugal cavity through the refrigeration component 211 attached to the outer side wall of the centrifugal bowl 21, so as to ensure that the temperature range of the centrifugal container for cell processing in the centrifugal cavity can be controlled, and the refrigeration speed is high, thus improving the refrigeration efficiency. At the same time, the cooling component 222 cools the heating surface of the refrigeration component 211, which can prevent the temperature of the refrigeration component 211 from rising with the refrigeration process, and prevent the influence of the temperature increase of the refrigeration component 211 on the refrigeration process, and ensure the temperature control stability and accuracy of the temperature control module 22 (the temperature control accuracy of the temperature control module 22 in this application is high, which can reach ±0.3°C; and the adjustable temperature range is accurate (6°C-37°C), which ensures the constant temperature required for cell processing. Moreover, the temperature control module 22 of the application has a simple structure and a small volume, which reduces the equipment cost.

Further, as shown in Figs. 8 and 9, the refrigeration component 211 includes a first semiconductor chilling plate 2211 with a chilling surface attached to the first side of the centrifugal bowl 21. The cooling component 222 includes a refrigeration fan 2221, a cooling plate 2222 connected to the heating surface of the first semiconductor cooling plate 2211, and a first air conduit 2223 connected between the cooling plate 2222 and the refrigeration fan 2221. That is, using first semiconductor chilling plate 2211 for refrigeration can make the refrigeration and heat dissipation structure simpler than that of water cooling conduit. In this embodiment, the chilling surface of the first semiconductor chilling plate 2211 is first attached to the outer side wall of the first side of the centrifugal bowl 21 through thermal conductive silicone grease. Specifically, the outer side wall of the first side of the centrifugal bowl 21 is provided with a first groove, attaching the first semiconductor chilling plate 2211 to the first groove can fully transfer the cooling capacity to the inside of the centrifugal cavity during the refrigeration process. Understandably, the cooling plate 2222 is attached to the heating surface of the first semiconductor cooling plate 2211 through thermal conductive silicone grease. The cooling plate 2222 presses the first semiconductor chilling plate 2211 and fixes the cooling plate 2222 on the outer side wall of the centrifugal bowl 21 by screws. Then the first air conduit 2223 is fixed on the cooling plate 2222 with screws, and finally the refrigeration fan 2221 is installed at the end of the first air conduit 2223 away from the cooling plate 2222, and then it is fixed with screws. In this way, after the first semiconductor chilling plate 2211 is electrified, the heating surface of the first semiconductor chilling plate 2211 discharges heat through the cooling plate 2222, first air conduit 2223 and refrigeration fan 2221.

Further, as shown in Figs. 8 and 9, the end of the first air conduit 2223 away from the refrigeration fan 2221 is provided with a mounting part 2224, the mounting part 2224 is provided with a positioning groove 2225 adapted to the cooling plate 2222, and the mounting part 2224 is installed on the outer side wall of the centrifugal bowl 21 through the cooling plate 2222 positioned in the positioning groove 2225. That is, the arrangement of the positioning groove 2225 allows the installation of the cooling plate 2222 in the positioning groove 2225, which can better protect the cooling plate 2222 and make the installation of the first air conduit 2223 more stable, and the refrigeration fan 2221 will have a better cooling effect on the cooling plate 2222 through the first air conduit 2223. The refrigeration component 211 further includes a second semiconductor chilling plate 2212 with a chilling surface attached to the second side of the centrifugal bowl 21. The first side and the second side are oppositely arranged. The cooling component 222 further includes a cooling fan 2226, a second air conduit 2227 and a heat conduit cooler 2228 connected to the heating surface of the second semiconductor cooling plate 2212. An end of the heat conduit cooler 2228 is connected with the cooling fan 2226, and the other end of the heat conduit cooler 2228 opposite to the cooling fan 2226 is connected with the air inlet of the second air conduit 2227. The refrigeration component 211 may also be attached to multiple sides of the same plane of the centrifugal bowl 21 to form a ring-shaped distribution, so that a closed refrigeration ring can be formed, which can make the temperature of the central point of the plane drop sharply and accelerate the refrigeration effect at the central point.

That is, in this embodiment, as shown in Figs. 8 and 9, the first semiconductor chilling plate 2211 and the second semiconductor chilling plate 2212 are symmetrically installed on the outer side wall of the centrifugal bowl 21, so that the cooling speed can be further improved. The chilling surface of the second semiconductor chilling plate 2212 is first attached to the outer side wall of the second side of the centrifugal bowl 21 through thermal conductive silicone grease. Specifically, a second groove is formed on the outer side wall of the second side of the centrifugal bowl 21. Attaching the second semiconductor chilling plate 2212 to the second groove allows the cooling capacity to be fully transferred to the inside of the centrifugal cavity during its refrigeration process. While the heat conduit cooler 2228 is attached to the heating surface of the second semiconductor chilling plate 2212 through thermal conductive silicone grease, and the heat conduit cooler 2228 presses the second semiconductor chilling plate 2212. The heat conduit cooler 2228 is fixed on the outer side wall of centrifugal bowl 21 by screws. A cooling fan 2226 is arranged above the heat conduit cooler 2228, and the second air conduit 2227 is fixed on the centrifugal bowl 21. And the air inlet of the second air conduit 2227 is positioned above the heat conduit cooler 2228 and opposite to the cooling fan 2226, so that the heat in the heat conduit cooler can be exhausted by the flowing wind using the cooling fan 2226.

In an embodiment, as shown in Figs. 8 and 9, the temperature control module 22 further includes a temperature sensor 224 installed at the bottom of the centrifugal cavity of the centrifugal bowl 21. The temperature sensor 224 is a high-precision temperature sensor 224, and the temperature sensor 224 is inserted into the centrifugal cavity and installed and fixed at the bottom of the centrifugal cavity for detecting the air temperature inside the centrifugal cavity in real time during cell processing. Understandably, the temperature sensor 224 can be fixed by thermal grease, and the temperature in the centrifugal bowl 21 monitored by the temperature sensor 224 in real time can be fed back to the control panel of the cell processing device. Further, the control panel controls the current of the refrigeration component 211 (such as the first semiconductor cooling plate 2211 and second semiconductor cooling plate 2212) according to the temperature, and further controls the temperature in the centrifugal bowl 21 to be kept within a preset range.

In an embodiment, as shown in Figs. 8 and 9, the bottom of the centrifugal bowl 21 is provided with a liquid discharge port (not shown). The centrifugal device 2 further comprises a liquid detection sensor 25 arranged at the bottom of the centrifugal cavity of the centrifugal bowl 21 (the liquid detection sensor 25 is fixed by sealant), and a liquid discharging connector 23 communicating with the liquid discharge port. Understandably, the liquid detection sensor 25 can monitor the liquid remaining in the centrifugal cavity. For example, when the liquid detection sensor 25 monitors that a certain amount of liquid appears at the bottom of the centrifugal cavity, the controller will trigger a prompt according to the received detection signal of the liquid detection sensor 25. Then, the controller can control the liquid discharging connector 23 to open, and then the liquid is discharged through the liquid discharge port and the liquid discharging connector 23. Further, the liquid discharging connector 23 can be externally connected with a drainage tube to discharge liquid (such as condensed water) in the centrifugal cavity through the liquid discharge port, the liquid discharge connector and the drainage tube, so as to prevent liquid accumulation or leakage.

In an embodiment, as shown in Figs. 1, 8 and 9, the edge of the centrifugal cavity of the centrifugal bowl 21 is provided with a connecting flange 212. The centrifugal device 2 further includes a sealing ring 24 attached to the connecting flange 212, and a flip cover 26 rotatably connected to the housing 5. The flip cover 26 is arranged at the edge of the centrifugal cavity and is used to cooperate with the sealing ring 24 to close or open the centrifugal cavity. That is, the sealing ring 24 is glued and fixed at the connecting flange on the top of the centrifugal bowl 21, so to cooperate with the flip cover 26 of the centrifugal device 2 to seal the centrifugal cavity, which ensures the safety and tightness of the cell processing and can also avoid the leakage of chill air. Before the cell processing device starts to work, the flip cover 26 can be opened, and after the liquid in the sample 4 is consumed (after all the liquid is injected into the centrifugal container for cell processing), operations such as conduit insertion and replacement of the centrifugal container need to be performed, and the flip cover 26 can be closed to fit the sealing ring 24 to seal the centrifugal cavity, which can play the role of safety protection and maintain the temperature in the centrifugal cavity.

In an embodiment, as shown in Fig. 1, the cell processing device further comprises a controller (not shown) installed in the housing 5, a code scanning device 6 for scanning code for recognition of the sample 4, a display device 7 for touch operation or parameter display. The weighing component 12, centrifugal device 2, liquid path module 3, code scanning device 6 and display device 7 are all connected to the controller. The display device 7 sets, modifies and monitors the relevant parameters in the cell processing process by touching the human-computer interaction display interface. The display device 7 can be rotatably connected to the housing 5 (e.g., the housing 5 is rotatably connected through a hinge), so as to facilitate storage and reduce the volume of the device. Moreover, the rotation angle of the display device 7 can be adjusted according to the visual angle requirements of the operator, thus improving the user experience. Understandably, the shape and size of the display device 7 can be designed as required. The code scanning device 6 is used to scan and identify the code on the sample 4, and then transmit the identified data information to the controller for use. The size and shape of the code scanning device 6 can also be set according to the requirements.

In an embodiment, as shown in Figs. 1 and 10. the conduit component 31 comprises a pinch valve 311, a bubble sensor 312, a pressure sensor 313, a peristaltic pump 314 and a stepping motor 315; the pinch valve 311 is used for controlling on-off of the conduit component's 31 conduit, the bubble sensor 312 is used for determining whether liquid in the sample 4 flows away by monitoring whether bubbles are mixed in the liquid flowing in the conduit in real time, the pressure sensor 313 is used for monitoring pressure of the liquid in the conduit in real time, the peristaltic pump 314 is used for driving the liquid in the conduit to flow during operation, an output end of the stepping motor 315 is connected with the peristaltic pump 314, and the stepping motor 315 is used for driving the peristaltic pump 314 to operate. The pinch valve 311, the bubble sensor 312, the pressure sensor 313 and the peristaltic pump 314 are all installed on a front face of the liquid path panel 32, the stepping motor 315 is installed on a back face of the liquid path panel 32 opposite to the peristaltic pump 314, and the stepping motor 315 is positioned in the housing 5. That is, the pinch valve 311, bubble sensor 312, pressure sensor 313 and peristaltic pump 314 are installed on the front of the liquid path panel 32 and fixed with screws. The stepping motor 315 is installed on the back of liquid path panel 32 and fixed with screws. The peristaltic pump 314 is installed above stepping motor 315 and on the front of liquid path panel 32, and fixed with screws. The conduit component 31 includes not only the above components, but also conduits connected between components. When the cell processing device is in the working state, the stepping motor 315 drives the peristaltic pump 314 to rotate, which is used as the power of the liquid flow in the sample 4 to drive the liquid flow. The pinch valve 311 (one or more can be provided as required, and the switch can be driven by electromagnetism) plays the role of switch, controlling the on-off of the liquid in the conduit used to convey the liquid in sample 4 and changing the flow path. The bubble sensor 312 (one or more can be provided as required) are used to monitor whether the fluid in the conduit is mixed with bubbles in real time. It is used as a criterion to determine whether the liquid in the sample 4 is used up. The pressure sensor 313 (one or more can be provided as required) monitors the liquid pressure in the conduit in real time. If the pressure exceeds the preset pressure threshold, the controller will send a feedback signal after receiving the pressure signal of the pressure sensor 313, and automatically control the conduit component 31 to adjust itself or notify the designated operator of the cell processing device to adjust. In this application, the conduit component 31 is laid out on the liquid path panel 32, and the liquid path panel 32 can be installed on the housing 5 at an inclination of 125°, which is convenient for operation, installation and disassembly. In addition, the liquid path panel 32 is equipped with a pressure sensor 313 to monitor the pressure of the liquid path to prevent conduit blockage. The liquid path panel 32 is also equipped with a bubble sensor 312 to monitor the air bubbles in the conduit for determining whether the liquid in the sample 4 is used up, which makes the use process more convenient.

In an embodiment, as shown in Fig. 1 and Fig. 10, after the start button 51 on the housing 5 of the cell processing device is pressed, it waits for a few seconds to start working. At this state, the relevant parameters can be set through the human-computer interaction display interface of display device 7. The code scanning device 6 is used to scan and identify the identification code on the sample 4, and the data information is sent to the controller to complete the data information input. Then, the pinch valve 311 on the liquid path panel 32 is opened through the human-computer interaction display interface of the display device 7, and the sample 4 is installed. The sample 4 is hung on the hook 123 of the corresponding weighing device 1. The conduit is connected with the pinch valve 311, pressure sensor 313 and bubble sensor 312 installed on liquid path panel 32, the centrifugal container is installed on the rotating mounting seat 106 of the centrifuge module 100, and the flip cover 26 is closed to close the centrifugal cavity. After checking that sample 4 is installed correctly, trigger the start button on the touch human-computer interaction display interface to start the cell processing of the liquid flowing into the centrifugal container in sample 4. Once the sample 4 is checked and installed correctly, the start button on the human-computer interaction display interface is triggered to start cell processing of the liquid flowing into the centrifugal container in sample 4. After the cell processing is completed, the pinch valve 311 on the liquid path panel 32 is opened, and the sample 4 is taken out to complete the work. The adjustable temperature range is accurate (4°C-40°C), and the temperature control accuracy is high (±0.3°C). moreover, the cell processing device has low power consumption, long service life and low noise.

The above are merely the preferred embodiments of the cell processing device of this application, and are not intended to limit the application. Any modification, equivalent substitution and improvement made within the spirit and principle of this application shall be included in the protection scope of this application.

## Claims

1. A cell processing device, comprising:
a weighing device, comprising a support component and at least one set of weighing components, wherein the support component comprises a bracket for supporting the weighing component; each set of weighing components comprises a mounting support seat installed on the bracket, a weighing sensor installed on the mounting support seat, and a hook detachably installed on the weighing sensor for hanging a sample;
a centrifugal device, comprises a centrifugal bowl with a centrifugal cavity, a centrifugal container arranged in the centrifugal cavity, and a centrifuge module connected with the centrifugal bowl and used for driving the centrifugal container to rotate;
a liquid path module, comprises a conduit component for conveying liquid in the sample into the centrifugal container for cell processing, and a liquid path panel for installing and controlling a liquid flow in the conduit component; and
a housing, wherein the centrifugal bowl, the liquid path panel and the bracket are all installed on the housing.

2. The cell processing device of claim 1, wherein there are at least two sets of the weighing components; the bracket comprises a mounting plate provided with mounting holes at intervals, and the number of the mounting holes is consistent with that of the weighing components; and
the mounting support seat is used to install the weighing sensor on the mounting plate at a position opposite to the mounting hole, and the hook passes through the mounting hole to connect the weighing sensor.

3. The cell processing device of claim 1, wherein the hook comprises a bending section, a lateral deflection section and a hitch section connected with the weighing sensor; opposite ends of the bending section are respectively connected with the hitch section and the lateral deflection section; the bending section and the lateral deflection section enclose to form a hook ring with an opening; and
the bending section and a central axis of the hitch section are both positioned on a first preset datum plane, and a central axis of the lateral deflection section and the first preset datum plane are arranged in a preset lateral deflection angle.

4. The cell processing device of claim 1, wherein the centrifuge module comprises a rotating shaft, a bearing component, a coupler, a drive motor for driving the rotating shaft to rotate, an actuator for actuating the rotating shaft, and a rotating mounting seat positioned in the centrifugal cavity and used for installing the centrifugal container; the coupler is connected between an output end of the drive motor and the rotating shaft; an end of the rotating shaft away from the coupler passes through the bearing component and is connected with the rotating mounting seat; and the actuator is connected with the bearing component.

5. The cell processing device of claim 4, wherein the bearing component comprises a bearing seat with a mounting through hole and two bearings installed on an inner side wall of the mounting through hole; the rotating shaft passes through the two bearings and is installed on the bearing seat; and the actuator is installed on the bearing seat; and
the centrifuge module further comprises an irregular wheel installed on the rotating shaft, and the actuator is provided with a clamping groove adapted to the irregular wheel.

6. The cell processing device of claim 1, wherein the centrifugal device further comprises a temperature control module for controlling the temperature of the centrifugal cavity; the temperature control module comprises a refrigeration component for refrigerating the centrifugal cavity and a cooling component for cooling the refrigeration component; a chilling surface of the refrigeration component is attached to an outer side wall of the centrifugal bowl, and a heating surface of the refrigeration component is connected with the cooling component.

7. The cell processing device of claim 6, wherein the refrigeration component comprises a first semiconductor chilling plate with a chilling surface attached to a first side of the centrifugal bowl; the cooling component comprises a refrigeration fan, a cooling plate connected with a heating surface of the first semiconductor cooling plate, and a first air conduit connected between the cooling plate and the refrigeration fan;
the refrigeration component further comprises a second semiconductor chilling plate with a chilling surface attached to a second side of the centrifugal bowl; the first side and the second side are oppositely arranged; and
the cooling component further comprises a cooling fan, a second air conduit and a heat conduit cooler connected with a heating surface of the second semiconductor chilling plate; an end of the heat conduit cooler is connected with the cooling fan, and another end of the heat conduit cooler opposite to the cooling fan is connected with an air inlet of the second air conduit.

8. The cell processing device of claim 1, wherein an edge of the centrifugal cavity of the centrifugal bowl is provided with a connecting flange; the centrifugal device further comprises a sealing ring attached to the connecting flange, and a flip cover rotatably connected to the housing; the flip cover is arranged at the edge of the centrifugal cavity and is used for cooperating with the sealing ring to close or open the centrifugal cavity.

9. The cell processing device of claim 1, wherein the cell processing device further comprises a controller installed in the housing, a code scanning device used for scanning code for recognition of the sample, a display device used for touch operation or parameter display, the weighing component, the centrifugal device, the liquid path module, the code scanning device and the display device are all connected with the controller.

10. The cell processing device of claim 1, wherein the conduit component comprises a pinch valve, a bubble sensor, a pressure sensor, a peristaltic pump and a stepping motor; the pinch valve is used for controlling on-off of the conduit component's conduit, the bubble sensor is used for determining whether liquid in the sample flows away by monitoring whether bubbles are mixed in the liquid flowing in the conduit in real time, the pressure sensor is used for monitoring pressure of the liquid in the conduit in real time, the peristaltic pump is used for driving the liquid in the conduit to flow during operation, an output end of the stepping motor is connected with the peristaltic pump, and the stepping motor is used for driving the peristaltic pump to operate; and
the pinch valve, the bubble sensor, the pressure sensor and the peristaltic pump are all installed on a front face of the liquid path panel, the stepping motor is installed on a back face of the liquid path panel opposite to the peristaltic pump, and the stepping motor is positioned in the housing.
